# EUROPEAN PATENT SPECIFICATION

(11) **EP 3 416 955 B1**
(45) Date of publication and mention of the grant of the patent: **09.09.2020**
(21) Application number: 17704274.4
(22) Date of filing: 14.02.2017
(51) Int. Cl.: C07D 317/54, A61K 31/343, A61P 13/04, A61P 13/12

(54) **USE OF STIRIPENTOL AND THEIR DERIVATIVES FOR DECREASING URINARY OXALATE CONCENTRATION IN AN INDIVIDUAL**
VERWENDUNG VON STIRIPENTOL UND DESSEN DERIVATE ZUR REDUZIERUNG DER URINOXALATKONZENTRATION IN EINEM INDIVIDUUM
USE OF STIRIPENTOL AND THEIR DERIVATIVES FOR DECREASING URINARY OXALATE CONCENTRATION IN AN INDIVIDUAL

(30) Priority: 15.02.2016 EP 16305173; 10.06.2016 EP 16305698
(43) Date of publication of application: 26.12.2018
(73) Proprietor: Institut National de la Santé et de la Recherche Médicale (INSERM), 75013 Paris (FR); Assistance Publique - Hôpitaux de Paris, 75004 Paris (FR); Sorbonne Université, 75006 Paris (FR)
(72) Inventor: LETAVERNIER, Emmanuel, 75020 Paris (FR); DAUDON, Michel, 75020 Paris (FR)
(74) Representative: Nony
(86) International application number: PCT/EP2017/053245
(87) International publication number: WO 2017/140658

(56) References cited:
- WO-A2-2008/058269
- WO-A2-2009/062118
- WO-A2-2011/159839
- Antônio Da Silva Novaes ET AL: "Diuretic and antilithiasic activities of ethanolic extract from Piper amalago (Piperaceae)", Phytomedicine, vol. 21, no. 4, 1 March 2014 (2014-03-01), pages 523-528, XP055262277, AMSTERDAM, NL ISSN: 0944-7113, DOI: 10.1016/j.phymed.2013.10.014

## Description

The present text describes compounds for their use to decrease urinary oxalate concentration in an individual and the present invention in particular relates to said compounds for their use in the prevention and/or treatment of diseases and/or conditions related to a high urinary oxalate concentration in an individual.

As used herein, the term "urinary oxalate concentration" means oxalic acid and/or oxalate concentration in an individual's urine. This term may also refer to the quantity of oxalic acid and/or oxalate urinary excretion in a 24-hour period.

An increased content of urinary oxalate is one of the major causes of calcium oxalate supersaturation that lead to the formation of calcium oxalate crystals and thus to kidney stones. Indeed, most of the kidney stones are made of calcium oxalate crystals.

Urinary oxalate may come from oxalate intakes in food such as spinach, rhubarb, chocolate or tea which are well known to contain high amounts of oxalate. However, it mostly originates from an endogenous production of oxalate by the liver notably due to the oxidation reaction of glyoxylate to oxalate by lactate dehydrogenase isoform 5 (LDH-5).

It has to be noticed that oxalate may also be produced by the metabolization of ethylene glycol that could be ingested accidentally.

Thus, many diseases and/or conditions, like urolithiasis, also called urinary stone disease, hyperoxaluria and nephrocalcinosis are related to a high urinary oxalate concentration. Some renal failures may also be related to a high urinary oxalate concentration.

Antônio da Silva Novaez et al. (Phytomedicine, vol. 21, no. 4, 2014, pages 523-528) evaluated the diuretic effects and antilithiatic activity of the ethanolic extract of *Piper amalago* and concluded that this extract probably has preventive properties to urolithiasis.

WO2008058269 describes a method of treating or preventing a disorder characterized by impaired protein trafficking, the method comprising administering to a subject in need thereof an effective amount of a compound selected from two different Tables, or a pharmaceutically acceptable derivative thereof. Said Tables represent several pages of various compounds among which piperine is mentioned. Moreover, the disorder is also selected from a long list of various disorders, among which hyperoxaluria is mentioned.

Similarly, WO2009062118 describes compounds and methods for modulating protein trafficking and treating or preventing disorders characterized by impaired protein trafficking. Among the various and numerous disorders listed in this document, hyperoxaluria is mentioned. The compounds listed on several pages of this document have various forms, regrouped under a general formula.

WO2011159839 describes methods of treating or preventing a condition characterized by abnormal tissue or organ levels of uric acid in an individual comprising administering to the individual an effective amount of a particular compound defined through a general formula. Among the listed corresponding conditions, urolithiasis is mentioned.

Hyperoxaluria is characterized by an urinary oxalate concentration above 0.25 mmol/L or urinary oxalate excretion above 0.25 mmol/24 hours (Daudon M., Ann Urol (Paris), 2005 Dec;39(6):209-31).

Regarding urolithiasis, it means a condition characterized by the presence of kidney stones in the kidney or in the urinary tract. This disease affects about 10 % of the population in western countries. Since a majority of urinary stones (75-80%) are made of calcium oxalate, the control of concentration of oxalate in urine is an important part of a medical treatment program to prevent stone formation or recurrence. Unfortunately, control of urinary oxalate concentration through the diet can produce only a partial effect, because dietary oxalate contribution to urinary oxalate is only 8 to 40%, with the rest of it synthesized endogenously, mainly in liver.

At last, primary hyperoxaluria is a genetic disorder resulting from the mutation of liver enzymes and inducing increased production of glyoxylate that is converted into oxalate by LDH-5 (Cochat P et al., Nephrol Dial Transplant. 2012 May;27(5): 1729-36).

Three forms of the disease coexist: Type I, Type II and Type III primary hyperoxaluria which are respectively caused by mutations in the AGXT gene, in the GRHPR gene and in the HOGA1 gene.

In this disease, high amounts of oxalate are therefore excreted in the urine, leading to recurrent stone disease and kidney failure in young patients. These patients often need combined kidney and liver allografts.

For obvious reasons, lowering urinary oxalate in these patients can alleviate the severity of stone disease and avoid costly transplantation procedures.

It is accordingly essential to have efficient compounds that are able to decrease urinary oxalate concentration and thus to be used in the prevention and/or treatment of diseases and/or conditions related to a high urinary oxalate concentration.

The present text describes a compound of formula (I): 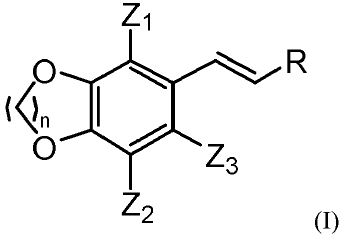 wherein:
- n is 1 or 2;
- Z₁, Z₂ and Z₃, identical or different, are selected from a hydrogen atom, a halogen atom and a (C₁-C₆)alkyl group; and
- R is an optionally substituted (C₁-C₆)alkyl group by one or more substituents chosen from a hydroxyl, an oxo and a thiol group;
or one of its pharmaceutically acceptable salts;
said compound of formula (I) being in all the possible racemic, enantiomeric and diastereoisomeric isomer forms;
for use in the prevention and/or treatment of diseases and/or conditions related to a high urinary oxalate concentration in an individual, said conditions being selected from hyperoxaluria, in particular primary hyperoxaluria, urolithiasis, nephrocalcinosis and some renal failures.

More particularly, according to one of its aspects, the present invention relates to a compound of formula (I): 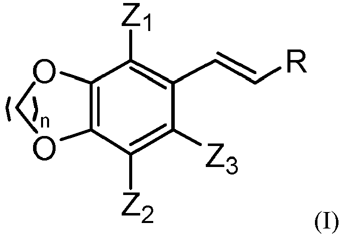 wherein:
- n is 1 or 2;
- Z₁, Z₂ and Z₃, identical or different, are selected from a hydrogen atom, a halogen atom and a (C₁-C₆)alkyl group; and
- R is an optionally substituted (C₁-C₆)alkyl group by one or more substituents chosen from a hydroxyl, an oxo and a thiol group;
or one of its pharmaceutically acceptable salts;
said compound of formula (I) being in all the possible racemic, enantiomeric and diastereoisomeric isomer forms;
for use in the prevention and/or treatment of diseases and/or conditions related to a high urinary oxalate concentration in an individual,
said diseases and/or conditions being selected from hyperoxaluria, in particular primary hyperoxaluria, urolithiasis, nephrocalcinosis and some renal failures.

Stiripentol, or 4,4-dimethyl-1-[(3,4-methylenedioxy)-phenyl]-1-penten-3-ol, is already proposed as an antiepileptic indicated in severe myoclonic epilepsy in infancy, in addition to the combination of sodium valproate and clobazam when the latter proves to be insufficient in controlling crises (Chiron et al. (2000) Lancet 356:1638-1642).

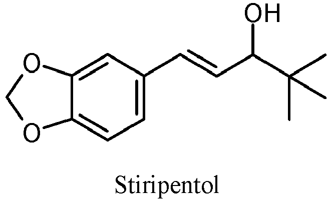

Among its main effects, Stiripentol inhibits the uptake of gamma-aminobutyric acid (GABA) and is also an inhibitor of several cytochrome P450 isoenzymes, especially CYP1A2 and CYP3A4 (Tran et al. (1997) Clin. Pharmacol. Ther. 62:490-504).

Stiripentol is also known for its use in the prevention or the treatment of peripheral neuropathy (EP 2 229 943 A1).

In particular, it has been shown that both Stiripentol and Isosafrole (3,4-methylenedioxyphenyl-1-propene) inhibit the pyruvate to lactate conversion by lactate dehydrogenase 1 and 5 (LDH-1 and LDH-5) in neurons (Sada N et al., Science 2015 Mar 20;347(6228): 1362-7).

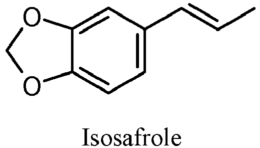

However, to the knowledge of the inventors, these compounds are proposed for the first time as active agent for lowering the content of urinary oxalate in an individual.

As shown in the following example, the inventors have unexpectedly identified that compounds of formula (I) enable to lower the content of urinary oxalate in an individual.

As a consequence, they are very efficient to limit calcium oxalate supersaturation and consequently the formation of kidney stones.

They are thus very useful for preventing or treating diseases characterized by a elevated urinary oxalate concentration such as urolithiasis and hyperoxaluria, more particularly primary hyperoxaluria.

For the purposes of the present text and invention:
- An "*individual*" is intended to mean an animal, preferably a mammal, including a non-human mammal, and is in particular a human.
- The term "*prevent*" means at least partly reducing the risk of manifestation of a given phenomenon, *i.e.,* in the present invention, a disease or a condition related to a high urinary oxalate concentration, said diseases and/or conditions being selected from hyperoxaluria, in particular primary hyperoxaluria, urolithiasis, nephrocalcinosis and some renal failures. A partial reduction implies that the risk remains, but to a lesser extent than before implementing the invention.
- A "*high urinary oxalate concentration*" or an "*elevated urinary oxalate content*" means an urinary oxalate concentration above 0.25 mmol/L, preferably above 0.3 mmol/L and more preferably above 0.5 mmol/L; or an urinary oxalate excretion above 0.25 mmol/24 hours, in particular above 0.3 mmol/24 hours and more particularly above 0.5 mmol/24 hours; or an oxalate/creatinine molar ratio above 0.03, preferably above 0.04 and more preferably above 0.05.
   It has to be noticed that these values correspond to those of adults or children of more than 5 years old. In younger children, urinary oxalate excretion is physiologically more elevated and decreases progressively during infancy.

The present text also describes a compound of formula (I): 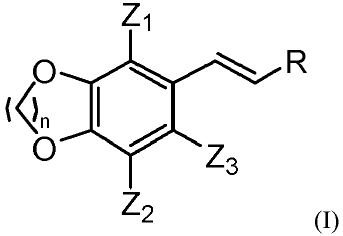 wherein:
- n is 1 or 2;
- Z₁, Z₂ and Z₃, identical or different, are selected from a hydrogen atom, a halogen atom and a (C₁-C₆)alkyl group; and
- R is an optionally substituted (C₁-C₆)alkyl group by one or more substituents chosen from a hydroxyl, an oxo and a thiol group;
or one of its pharmaceutically acceptable salts;
said compound of formula (I) being in all the possible racemic, enantiomeric and diastereoisomeric isomer forms;
for use to decrease urinary oxalate concentration in an individual.

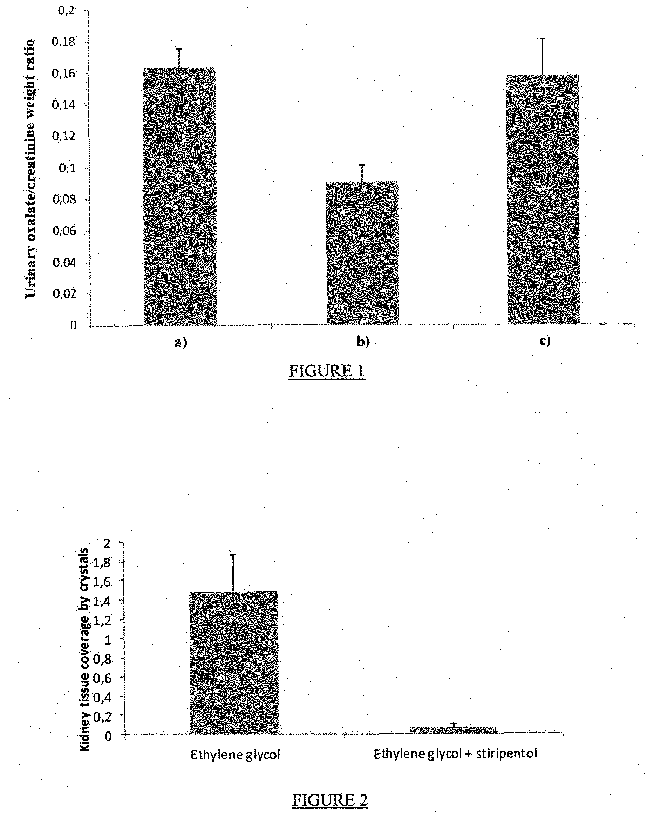

Other features and advantages of the invention will emerge more clearly from the description and from the figure 1.
Figure 1 represents a graph that gives the average urinary oxalate/creatinine molar ratio measured in urine from 6 rats in cases a) (before the administration of Stiripentol), b) (after the two-days intake of Stiripentol at a daily dose of 100 mg/kg of rat) and c) (ten days after the two-day intake of Stiripentol).
Figure 2 represents a graph that gives the percentages of kidney section coverage by calcium oxalate crystals measured in rats exposed to ethylene glycol and that have been or not treated with Stiripentol.
Figure 3 represents a graph that shows the benefit effect of Stiripentol on the renal function assessed by serum creatinine levels observed on rats intoxicated with ethylene glycol.
Figure 4 represents a graph that shows the benefit effect of Stiripentol on the preserved renal function assessed by serum creatinine levels observed on calcium-oxalate kidney stone murine.

### COMPOUNDS OF FORMULA (I)

As above-mentioned, the compounds used according to the invention, i.e. the compounds for use in the prevention and/or treatment of diseases and/or conditions related to a high urinary oxalate concentration in an individual, said diseases and/or conditions being selected from hyperoxaluria, in particular primary hyperoxaluria, urolithiasis, nephrocalcinosis and some renal failures, correspond to general formula (I): 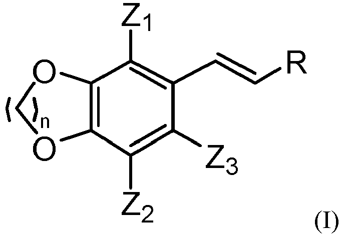 wherein:
- n is 1 or 2;
- Z₁, Z₂ and Z₃, identical or different, are selected from a hydrogen atom, a halogen atom and a (C₁-C₆)alkyl group; and
- R is an optionally substituted (C₁-C₆)alkyl group by one or more substituents chosen from a hydroxyl, an oxo and a thiol group;
or one of its pharmaceutically acceptable salts;
said compound of formula (I) being in all the possible racemic, enantiomeric and diastereoisomeric isomer forms.

Accordingly, the compounds of the invention may comprise one or several asymmetric carbon atoms. They thus may exist in the form of enantiomers or diastereoisomers. These enantiomers and diastereoisomers, and also mixtures thereof, including racemic mixtures, form part of the invention. Further, the alkene function that is present in the compound of formula (I) can either be in the Z or in the E configuration, preferably, this alkene is in the E configuration.

The compounds of the invention may also exist in the form of bases or of acid-addition salts. These salts are pharmaceutically acceptable acids and also form part of the invention.

The term "*pharmaceutically acceptable*" means what is useful in preparing a pharmaceutical composition generally safe, non-toxic, and neither biologically nor otherwise undesirable and includes what is acceptable for veterinary as well as human pharmaceutical use.

In addition to pharmaceutically-acceptable salts, other salts are included in the invention. They may serve as intermediates in the purification of the compounds, in the preparation of other salts, or in the identification and characterization of the compounds or intermediates.

The compounds of the invention may also exist in the form of hydrates or solvates, *i.e.* in the form of associations or combinations with one or more molecules of water or with a solvent such as methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

In the context of the present invention, the following definitions apply:
- a halogen atom: a fluorine, a chlorine, a bromine or an iodine atom.
- Cₜ-C_{z}: a carbon-based chain possibly containing from t to z carbon atoms in which t and z may take values from 1 to 10; for example, C₁-C₃ is a carbon-based chain possibly containing from 1 to 3 carbon atoms.
- an alkyl: a linear or branched saturated aliphatic group, in particular comprising form 1 to 6 carbon atoms. Examples that may be mentioned include methyl, ethyl, n-propyl, isopropyl, butyl, isobutyl, tert-butyl, pentyl, neopenthyl. The alkyl groups may be more particularly selected from methyl and neopenthyl groups.
- a hydroxyl group: a radical -OH.
- an oxo group: a radical =O.
- a thiol group: a radical -SH.

According to a preferred embodiment, n is 1 in the compound used in the invention.

According to another preferred embodiment, at least one from Z₁, Z₂ and Z₃ is a hydrogen atom, in the compound used in the invention.

More preferably Z₁, Z₂ and Z₃ are hydrogen atoms.

According to yet another preferred embodiment, the compound used in the invention is selected from 4,4-dimethyl-1-[3,4(methylenedioxy)-phenyl]-1-penten-3-ol (Stiripentol), 3,4-methylenedioxyphenyl-1-propene (Isosafrole) and their mixtures.

The four following stereoisomers of Stiripentol exist depending of the absolute configuration of the asymmetric carbon and of the alkene function:

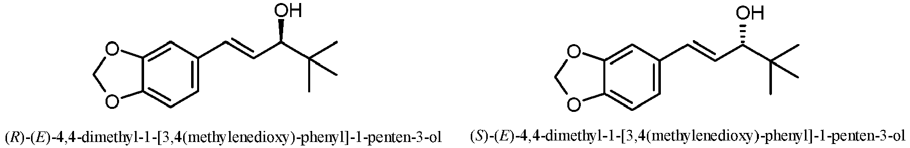

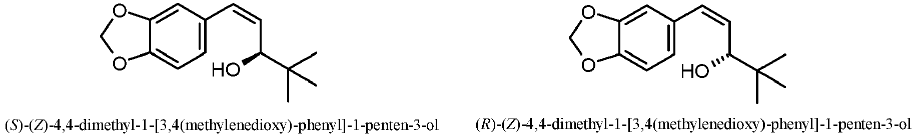

The two following stereoisomers of Isosafrole exist depending of the absolute configuration of the alkene function:

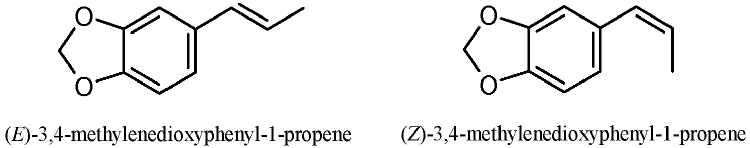

For both Stiripentol and Isosafrole, the alkene is preferably in the (E) configuration.

Concerning Stiripentol, it is preferably in the form of a racemic mixture of (*R*) and (*S*) enantiomer.

Thus, the compound used in the invention is more particularly selected from (*RS*)-(*E*)-4,4-dimethyl-1-[3,4(methylenedioxy)-phenyl]-1-penten-3-ol, (*E*)-3,4-methylenedioxyphenyl-1-propene and their mixtures.

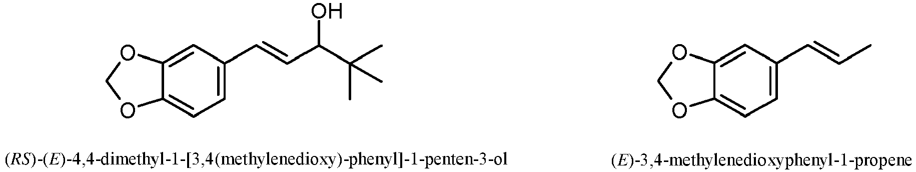

FR 2 173 691 describes the synthesis of Stiripentol starting from pinacoline and piperonal.

The skilled artisan is able to easily prepare the other compounds used in the invention starting from the teaching of FR 2 173 691 and by using methods well-known by the man skilled in the art.

Some compounds used in the invention are commercially available. Stiripentol (in the form of a racemic mixture with the alkene is in the (E) configuration) for instance, may be provided under the name Diacomit® sold by Laboratoires Biocodex.

### THERAPEUTIC USE

As specified previously and clearly illustrated by the following example, according to one of its aspects, the present text describes a compound of the present invention for use to decrease urinary oxalate concentration in an individual.

In other terms, the present text describes the use of a compound of the present invention to decrease urinary oxalate concentration in an individual.

In particular, as shown in the following example, some compounds of the invention like Stiripentol enable to reduce the urinary oxalate/creatinine molar ratio of an individual by at least 10%, preferably by at least 20%, more preferably by at least 30 % and even more preferably by at least 40 %.

The invention relates to a compound of the present invention for use in the prevention and/or the treatment of diseases and/or conditions related to a high urinary oxalate concentration in an individual, said diseases and/or conditions being selected from hyperoxaluria, in particular primary hyperoxaluria, urolithiasis, nephrocalcinosis and some renal failuress.

The text describes the use of a compound of the present invention to prevent and/or treat diseases related to a high urinary oxalate concentration in an individual.

In yet other words, the text describes the use of a compound of the present invention for the preparation of a medicament intended to prevent and/or to treat diseases and/or conditions related to a high urinary oxalate concentration in an individual.

In particular, said diseases and/or conditions may be selected from:
- hyperoxaluria, in particular primary hyperoxaluria, preferably Type I, Type II or Type III primary hyperoxaluria,
- urolithiasis also called urinary stone disease in particular due to primary hyperoxaluria,
- nephrocalcinosis in particular due to primary hyperoxaluria, and
- some renal failures, i.e. renal failures related to a high urinary oxalate concentration in an individual.

The compound according to the invention may also be useful to prevent and/or treat troubles caused by oxalate precipitation due to ethylene glycol poisoning.

The text also described a method for preventing and/or treating diseases and/or conditions related to a high urinary oxalate concentration in an individual, comprising at least a step of administering to said individual at least an effective amount of at least one compound in accordance with the invention as above defined.

The compound according to the invention may be used in a composition comprising a pharmaceutically acceptable excipient.

Such a composition is considered as a pharmaceutical composition or as a medicament and may more particularly contain an effective dose of at least one compound according to the invention as above defined.

An "*effective dose*" means an amount sufficient to induce a positive modification in the condition to be regulated or treated, but low enough to avoid serious side effects. An effective dose may vary with the pharmaceutical effect to obtain or with the particular condition being treated, the age and physical condition of the end user, the severity of the condition being treated/prevented, the duration of the treatment, the nature of other treatments, the specific compound or composition employed, the route of administration, and like factors.

A compound used according to the invention may be administered in an effective dose by any of the accepted modes of administration in the art.

In one embodiment, this compound may be used in a composition intended to be administrated by oral, rectal or parenteral injection route, preferably by oral route, in particular diluted in a drink such as water.

*The term "parenteral injection"* refers to an administration via injection under or through one or more layers of skin or mucus membranes of an individual. This injection may be for instance intradermal, subcutaneous, intravenous or intramuscular.

The route of administration and the galenic formulation will be adapted by one skilled in the art pursuant to the desired pharmaceutical effect.

One of ordinary skill in the art of therapeutic formulations will be able, without undue experimentation and in reliance upon personal knowledge, to ascertain a therapeutically effective dose of a compound of the invention for a given indication.

The compound of the invention may be administered to a subject once a week, twice a week, four times a week, once a day, twice a day, three times a day or more if necessary, and such administration can be for one day, two days, three days, four days, five days, or a week, two weeks, three weeks, a month, two months, four months, six months, more than six months, one year, two years, or continuously through the life of the patient. Such treatment may be continued to maintain the desired oxalate levels in a subject.

The daily dose may ranges from 20 to 150, preferably from 50 to 100 mg of compound of formula (I) or of its pharmaceutically acceptable salt/kg of the treated individual.

According to a preferred embodiment, the composition used in the invention is suitable for administering a daily dose comprising from 20 to 150, preferably from 50 to 100 mg of compound of formula (I) or of its pharmaceutically acceptable salt/kg of said individual, for a period of at least 1 day.

It has to be noted that such a daily dose may as well be administered for a period of 2 days, 1 week, 1 month, several months, one year and even for a longer duration.

A pharmaceutical composition of the invention may be formulated with any known suitable pharmaceutically acceptable excipients according to the dose, the galenic form, the route of administration and the likes.

As used herein, "*pharmaceutically acceptable excipients*" include any and all solvents, dispersion media, coatings, antibacterial and antifungal agents, isotonic and absorption delaying agents and the like. Except insofar as any conventional excipient is incompatible with the active compounds, its use in a medicament or pharmaceutical composition of the invention is contemplated.

A medicament or pharmaceutical composition of the invention may be in the form of tablets, pills, powders, lozenges, sachets, cachets, elixirs, suspensions, emulsions, solutions, syrups, aerosols, sprays, gels, soft and hard gelatine capsules, suppositories, sterile injectable solutions or sterile packages powders.

According to one embodiment, a pharmaceutical composition of the invention may be intended to be administered separately, sequentially or simultaneously with an agent useful for the prevention and/or the treatment of a disease and/or condition related to a high urinary oxalate concentration, in particular hyperoxaluria, nephrocalcinosis, urolithiasis or some renal failures said agent being different from the compound of formula (I) of the invention and its pharmaceutically acceptable salts.

Among such agents the following ones may be cited: pyridoxamine, inhibitors of aldehyde dehydrogenase, inhibitors of glycolate oxidase; or in combination with therapies such as extracorporeal shock wave lithotripsy and reduced oxalate level diets. As used herein, the term "reduced oxalate level diet" means diet from which oxalate-rich foods (rhubarb, spinach and other leafy vegetables, cashews, almonds, and strong tea) have been eliminated.

### EXAMPLE 1: Stiripentol assay (in vivo)

The efficiency of Stiripentol to decrease the urinary oxalate concentration in rats has been evaluated in this example.

### Protocol

The experiment has been performed on 6 adult Sprague-Dawley rats that have not previously been treated.

100 mg/kg of rat of Stiripentol (Diacomit® sold by Laboratoires Biocodex) has been administered to the rats by oral route in drinking water over a period of 24 hours. This protocol has been repeated the next day.

Rats had a free access to water during the experiment.

In order to obtain more reliable data, the urinary oxalate/creatinine molar ratio has been measured instead of the urinary oxalate concentration. Indeed, the content of creatinine, which is a metabolic waste of creatine excreted in the urine, is constant over 24 hours for an individual, whereas the urinary oxalate concentration may varies depending on the quantity of drink consumption. The urinary oxalate/creatinine molar ratio measurement thus enables to overcome the problem of dilution of the urines.

This ratio has been measured in the urine of the 6 rats by using metabolic cages (commercialized by Techniplast) that collect urine during 24 hours. More particularly, the oxalate urine level has been measured by chromatography with a column commercialized by Dionex and the urine creatinine level has been measured by an enzymatic method with a Konelab apparatus.

For the 6 rats, the urinary oxalate/creatinine molar ratio has been measured three times in the urine collected in the metabolic cages:
- a) before the administration of Stiripentol,
- b) after the two-days intake of Stiripentol, and
- c) ten days after the two-day intake of Stiripentol.

The urinary calcium/creatinine molar ratio has been measured for the 6 rats in cases a), b) and c) in the same way as the urinary oxalate/creatinine molar ratio to ensure that Stiripentol affects oxalate concentration and not creatinine concentration in the oxalate/creatinine molar ratio (control measurements).

### Statistical analysis

Statistical analysis was done using unpaired Student t test and Mann Whitney test.

A p value of less than 0.05 was considered as significant.

Statistical analysis was completed using Statview software

### Results

The average urinary oxalate/creatinine molar ratios obtained for the 6 rats in cases a), b) and c) are presented in Figure 1.

It can be seen that measures a) and c) gave the same result, *i.e.* an urinary oxalate/creatinine molar ratio of about 0,163, whereas measure b) gave a ratio of about 0,093 (p=0.03).

Regarding the control measurements (not represented in Figure 1), the intake of Stiripentol has shown no effect on the urinary calcium/creatinine molar ratio.

It thus appears that Stiripentol is able to significantly reduce the urinary oxalate concentration and therefore to decrease calcium oxalate supersaturation and the risk of calcium oxalate stone formation.

Stiripentol is thus suitable for the prevention and/or treatment of a disease and/or condition related to a high urinary oxalate concentration such as primary hyperoxaluria, urolithiasis, nephrocalcinosis and some renal failures.

### EXAMPLE 2

Efficiency of Stiripentol to protect against ethylene glycol intoxication has been evaluated in this example.

A murine model of ethylene glycol poisoning has been prepared by giving 6g/kg ethylene glycol orally once to twelve adult Sprague-Dawley rats (single oral administration).

Among these rats, 6 received in addition 300 mg/kg Stiripentol (Diacomit®) at the time of the intoxication and in drinking water at day one and day 2 after intoxication (4g/L). Rats had a free access to water during the experiments.

Rats have been sacrificed 48 hours after ethylene glycol intake, blood and urine parameters have been collected and kidneys have been removed for histopathological analyses.

The histopathological analyses of kidneys from rats exposed to ethylene glycol show that these kidneys were full of calcium oxalate crystals whereas those receiving Stiripentol in addition contained much less crystals (Figure 2, p=0.004).

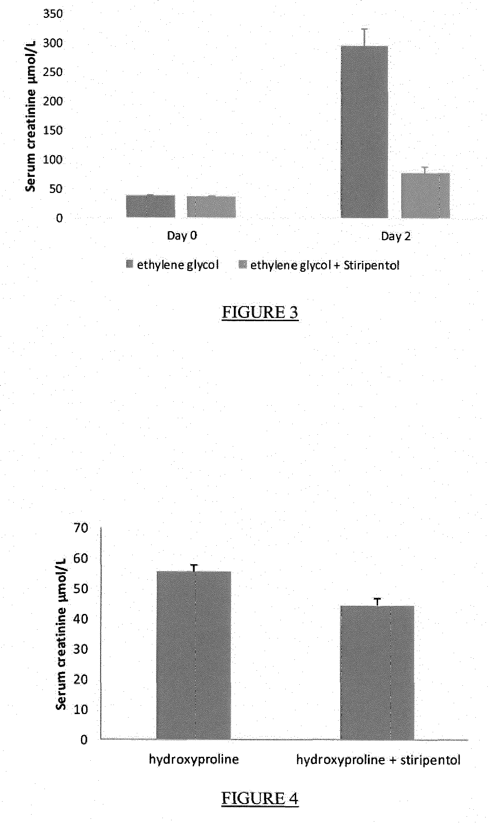

Renal function assessed by serum creatinine levels was remarkably less impaired in animals receiving stiripentol (Figure 3, p=0.009).

Accordingly, these results show that Stiripentol at therapeutic range (and by extent isosafrole) decreases urinary oxalate excretion induced by ethylene glycol poisoning and therefore decreases calcium oxalate supersaturation and the risks of calcium oxalate crystals formation and renal failure.

### EXAMPLE 3

Efficiency of Stiripentol to protect against calcium oxalate kidney stones has been evaluated in this example.

A calcium-oxalate kidney stone murine model has been generated. Twelve adult Sprague-Dawley rats received calcium chloride (2 g/1) and hydroxyproline (20g/L), an aminoacid transformed into oxalate by liver enzymes including LDH-5, in drinking water.

The rats have been exposed for 4 month to this diet.

Among these rats, 6 received in addition Stiripentol (Diacomit®) in drinking water at 4g/L, corresponding to about 150 mg/Kg in adult rats.

Rats have been sacrificed at the end of the protocol and kidneys have been removed for analyses.

Rats receiving Stiripentol had less calcium oxalate crystals in urine and a preserved renal function assessed by serum creatinine levels when compared to controls at the end of the 4-months protocol (Figure 4; p=0.002).

Thus, stiripentol is clearly efficient to prevent calcium oxalate stone formation, particularly in cases at risk of renal failure such as patients affected by primary hyperoxaluria and more generally in kidney stone formers affected by hyperoxaluria.

## Claims

1. Compound of formula (I) : 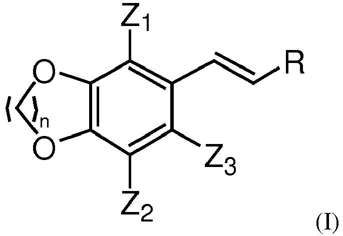 wherein:
- n is 1 or 2;
- Z₁, Z₂ and Z₃, identical or different, are selected from a hydrogen atom, a halogen atom and a (C₁-C₆)alkyl group; and
- R is an optionally substituted (C₁-C₆)alkyl group by one or more substituents chosen from a hydroxyl, an oxo and a thiol group;
or one of its pharmaceutically acceptable salts;
said compound of formula (I) being in all the possible racemic, enantiomeric and diastereoisomeric isomer forms;
for use in the prevention and/or treatment of diseases and/or conditions related to a high urinary oxalate concentration in an individual,
said diseases and/or conditions being selected from hyperoxaluria, in particular primary hyperoxaluria, urolithiasis, nephrocalcinosis and some renal failures.

2. Compound for use of formula (I) or one of its pharmaceutically acceptable salts according to claim 1, wherein n is 1.

3. Compound for use of formula (I) or one of its pharmaceutically acceptable salts according to claim 1 or 2, wherein at least one from Z₁, Z₂ and Z₃ is a hydrogen atom, preferably Z₁, Z₂ and Z₃ are hydrogen atoms.

4. Compound for use of formula (I) or one of its pharmaceutically acceptable salts according to any one of the preceding claims, selected from 4,4-dimethyl-1-[3,4(methylenedioxy)-phenyl]-1-penten-3-ol (Stiripentol), 3,4-methylenedioxyphenyl-1-propene (Isosafrole) and their mixtures, preferably selected from (*RS*)-(*E*)-4,4-dimethyl-1-[3,4(methylenedioxy)-phenyl]-1-penten-3-ol,(*E*)-3,4-methylenedioxyphenyl-1-propene and their mixtures.

5. Compound for use of formula (I) or one of its pharmaceutically acceptable salts according to any one of the preceding claims, wherein the individual is a mammal, in particular a human.

6. Compound for use of formula (I) or one of its pharmaceutically acceptable salts according to any one of the preceding claims, wherein it is in a composition comprising a pharmaceutically acceptable excipient.

7. Compound for use of formula (I) or one of its pharmaceutically acceptable salts according to claim 6, wherein the composition is administered by oral, rectal or parenteral injection route, preferably by oral route.

8. Compound for use of formula (I) or one of its pharmaceutically acceptable salts according to claim 6 or 7, wherein the composition is in the form of tablets, pills, powders, lozenges, sachets, cachets, elixirs, suspensions, emulsions, solutions, syrups, aerosols, sprays, gels, soft and hard gelatine capsules, suppositories, sterile injectable solutions or sterile packages powders.

9. Compound for use of formula (I) or one of its pharmaceutically acceptable salts according to any one of claims 6 or 8, wherein the composition is suitable for administering a daily dose comprising from 20 to 150, preferably from 50 to 100 mg of compound of formula (I) or of its pharmaceutically acceptable salt/kg of said individual, for a period of at least 1 day.

## Patentansprüche

1. Verbindung der Formel (I): 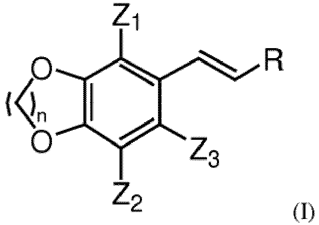 wobei:
- n für 1 oder 2 steht;
- Z₁, Z₂ und Z₃ gleich oder verschieden sind und aus einem Wasserstoffatom, einem Halogenatom und einer (C₁-C₆)-Alkylgruppe ausgewählt sind und
- R für eine (C₁-C₆)-Alkylgruppe, die gegebenenfalls durch einen oder mehrere Substituenten, die aus einer Hydroxyl-, einer oxo- und einer Thiolgruppe ausgewählt sind, substituiert ist, steht;
oder eines ihrer pharmazeutisch unbedenklichen Salze;
wobei die Verbindung der Formel (I) in allen der möglichen racemischen, enantiomeren und diastereoisomeren Isomerformen vorliegen;
zur Verwendung bei der Prävention und/oder Behandlung von Erkrankungen und/oder Leiden, die mit einer hohen Harnoxalatkonzentration bei einem Individuum zusammenhängen,
wobei die Erkrankungen und/oder Leiden aus Hyperoxalurie, insbesondere primärer Hyperoxalurie, Urolithiasis, Nephrokalzinose und einigen Nierenversagen ausgewählt ist.

2. Verbindung zur Verwendung der Formel (I) oder eines ihrer pharmazeutisch unbedenklichen Salze nach Anspruch 1, wobei n für 1 steht.

3. Verbindung zur Verwendung der Formel (I) oder eines ihrer pharmazeutisch unbedenklichen Salze nach Anspruch 1 oder 2, wobei mindestens eines von Z₁, Z₂ und Z₃ für ein Wasserstoffatom steht und vorzugsweise Z₁, Z₂ und Z₃ für Wasserstoffatome stehen.

4. Verbindung zur Verwendung der Formel (I) oder eines ihrer pharmazeutisch unbedenklichen Salze nach einem der vorhergehenden Ansprüche, ausgewählt aus 4,4-Dimethyl-1-[3,4(methylendioxy)-phenyl]-l-penten-3-ol (Stiripentol), 3,4-Methylendioxyphenyl-1-propen (Isosafrol) und Mischungen davon, vorzugsweise ausgewählt aus (*RS*)-(*E*)-4,4-Dimethyl-1-[3,4(methylendioxy)phenyl]-1-penten-3-ol, (*E*)-3,4-Methylendioxyphenyl-1-propen und Mischungen davon.

5. Verbindung zur Verwendung der Formel (I) oder eines ihrer pharmazeutisch unbedenklichen Salze nach einem der vorhergehenden Ansprüche, wobei es sich bei dem Individuum um ein Säugetier, insbesondere einen Menschen, handelt.

6. Verbindung zur Verwendung der Formel (I) oder eines ihrer pharmazeutisch unbedenklichen Salze nach einem der vorhergehenden Ansprüche, wobei sie bzw. es in einer Zusammensetzung, die einen pharmazeutisch unbedenklichen Hilfsstoff umfasst, vorliegt.

7. Verbindung zur Verwendung der Formel (I) oder eines ihrer pharmazeutisch unbedenklichen Salze nach Anspruch 6, wobei die Zusammensetzung oral, rektal oder durch parenterale Injektion, vorzugsweise oral, verabreicht wird.

8. Verbindung zur Verwendung der Formel (I) oder eines ihrer pharmazeutisch unbedenklichen Salze nach Anspruch 6 oder 7, wobei die Zusammensetzung in Form von Tabletten, Pillen, Pulvern, Pastillen, Beuteln, Oblatenkapseln, Elixieren, Suspensionen, Emulsionen, Lösungen, Sirupen, Aerosolen, Sprays, Gelen, Weich- und Hartgelatinekapseln, Zäpfchen, sterilen Injektionslösungen oder sterilen abgepackten Pulvern vorliegt.

9. Verbindung zur Verwendung der Formel (I) oder eines ihrer pharmazeutisch unbedenklichen Salze nach einem der Ansprüche 6 oder 8, wobei die Zusammensetzung zur Verabreichung einer Tagesdosis, die 20 bis 150 und vorzugsweise 50 bis 100 mg Verbindung der Formel (I) oder pharmazeutisch unbedenkliches Salz davon/kg des Individuums umfasst, über einen Zeitraum von mindestens 1 Tag geeignet ist.

## Revendications

1. Composé de formule (I) : 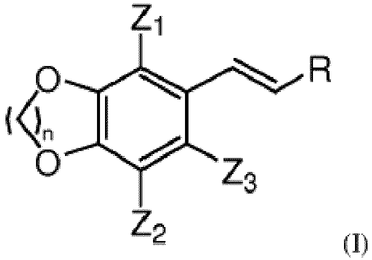
- n étant 1 ou 2 ;
- Z₁, Z₂ et Z₃, identiques ou différents, étant choisis parmi un atome d'hydrogène, un atome d'halogène et un groupe C₁₋₆-alkyle ; et
- R étant un groupe C₁₋₆-alkyle éventuellement substitué par un ou plusieurs substituants choisis parmi un groupe hydroxyle, un groupe oxo et un groupe thiol ;
ou un de ses sels pharmaceutiquement acceptables ; ledit composé de formule (I) étant dans toutes les formes isomères racémiques, énantiomériques et diastéréoisomériques possibles ;
pour une utilisation dans la prévention et/ou le traitement de maladies et/ou d'affections liées à une concentration urinaire élevée en oxalate chez un individu,
lesdites maladies et/ou affections étant choisies parmi l'hyperoxalurie, en particulier l'hyperoxalurie primaire, l'urolithiase, la néphrocalcinose et certaines insuffisances rénales.

2. Composé pour une utilisation de formule (I) ou un de ses sels pharmaceutiquement acceptables selon la revendication 1, n étant 1.

3. Composé pour une utilisation de formule (I) ou un de ses sels pharmaceutiquement acceptables selon la revendication 1 ou 2, au moins l'un parmi Z₁, Z₂ et Z₃ étant un atome d'hydrogène, préférablement Z₁, Z₂ et Z₃ étant des atomes d'hydrogène.

4. Composé pour une utilisation de formule (I) ou un de ses sels pharmaceutiquement acceptables selon l'une quelconque des revendications précédentes, choisi parmi le 4,4-diméthyl-1-[3,4 (méthylènedioxy)-phényl]-1-pentén-3-ol (Stiripentol), le 3,4-méthylènedioxyphényl-1-propène (Isosafrole) et leurs mélanges, préférablement choisi parmi le (*RS*)-(*E*)-4,4-diméthyl-1-[3,4(méthylènedioxy)-phényl]-1-pentén-3-ol, le (*E*)-3,4-méthylènedioxyphényl-1-propène et leurs mélanges.

5. Composé pour une utilisation de formule (I) ou un de ses sels pharmaceutiquement acceptables selon l'une quelconque des revendications précédentes, l'individu étant un mammifère, en particulier un humain.

6. Composé pour une utilisation de formule (I) ou un de ses sels pharmaceutiquement acceptables selon l'une quelconque des revendications précédentes, qui se trouve dans une composition comprenant un excipient pharmaceutiquement acceptable.

7. Composé pour une utilisation de formule (I) ou un de ses sels pharmaceutiquement acceptables selon la revendication 6, la composition étant administrée par voie orale, rectale ou d'injection parentérale, préférablement par voie orale.

8. Composé pour une utilisation de formule (I) ou un de ses sels pharmaceutiquement acceptables selon la revendication 6 ou 7, la composition se trouvant sous forme de comprimés, de pilules, de poudres, de pastilles, de sachets, de cachets, d'élixirs, de suspensions, d'émulsions, de solutions, de sirops, d'aérosols, de sprays, de gels, de capsules de gélatine souples et dures, de suppositoires, de solutions injectables stériles ou de poudres en emballages stériles.

9. Composé pour une utilisation de formule (I) ou un de ses sels pharmaceutiquement acceptables selon l'une quelconque des revendications 6 et 8, la composition étant appropriée pour l'administration d'une dose quotidienne comprenant de 20 à 150, préférablement de 50 à 100 mg du composé de formule (I) ou de ses sels pharmaceutiquement acceptables/kg dudit individu, pendant une période d'au moins 1 jour.
